# EUROPÄISCHE PATENTSCHRIFT

(11) **EP 1 482 847 B1**
(45) Veröffentlichungstag und Bekanntmachung des Hinweises auf die Patenterteilung: **05.08.2009**
(21) Anmeldenummer: 03707887.0
(22) Anmeldetag: 11.03.2003
(51) Int. Cl.: A61B 17/68

(54) **IMPLANTAT ZUR DYNAMISCHEN FIXATION EINER KORREKTUROSTEOTOMIE**
IMPLANT FOR THE DYNAMIC FIXATION OF A CORRECTIVE OSTEOTOMY
IMPLANT PERMETTANT LA FIXATION DYNAMIQUE D'UNE OSTEOTOMIE CORRECTRICE

(30) Priorität: 12.03.2002 AT 3822002
(43) Veröffentlichungstag der Anmeldung: 08.12.2004
(73) Patentinhaber: Frank, Erich, 1230 Wien (AT)
(72) Erfinder: Frank, Erich, 1230 Wien (AT)
(74) Vertreter: Miksovsky, Alexander
(86) Internationale Anmeldenummer: PCT/AT2003/000067
(87) Internationale Veröffentlichungsnummer: WO 2003/075775

(56) Entgegenhaltungen:
- EP-A- 1 287 787
- FR-A- 2 572 274
- GB-A- 1 274 470
- US-A- 5 603 715

## Beschreibung

Die vorliegende Erfindung betrifft ein Implantat zur dynamischen Fixation einer Korrekturosteotomie, wobei ein Befestigungsabschnitt mit einem Loch zur Aufnahme einer Spongiosaschraube über eine Kröpfung mit einem insbesondere seitlich versetzten, langgestreckten Verankerungsabschnitt verbunden ist, der in Längsrichtung als steifer Verankerungsschaft ausgebildet ist.

Ein beispielhaftes, spezielles Anwendungsgebiet solcher Implantate ist die Korrekturosteotomie zur Behandlung von Achsenfehlstellungen an Mittelfußknochen des Menschen, wie beispielsweise Hallux valgus.

Aus der WO 97/35528 ist ein als V-förmige Spange ausgebildetes Implantat zur Behandlung einer Achsenfehlstellung eines Mittelfußknochens bekannt. Diese Spange erlaubt keine völlig rotationsstabile Fixierung der zu verbindenden Knochenfragmente. Dadurch ergeben sich unter anderem Nachteile bei der Belastung des Fußes. Weiters ist das Einsetzen der Spange schwierig, da das zu verschiebende Knochenfragment meist unter Sehnenspannung steht. Außerdem kann die Spange beim Explantieren brechen.

Aus der US-A 5 603 715 ist ein Implantat der eingangs genannten Art bekannt geworden, wobei darauf abgezielt wird, sowohl eine statische als auch eine dynamische Verriegelung für die Behandlung von schräg bzw. quer verlaufenden Knochenbrüchen zur Verfügung zu stellen. Darüber hinaus ist aus der FR-A 2 572 274 ein Osteosynthesenagel bekannt geworden, welcher aus einem metallischen Blech oder durch Gießen aus einem Kunststoff hoher Festigkeit hergestellt werden kann.

Aufgabe der vorliegenden Erfindung ist es, ein verbessertes Implantat zur dynamischen Fixation einer Korrekturosteotomie anzugeben.

Zur Lösung dieser Aufgabe ist ein Implantat zur dynamischen Fixation einer Korrekturosteotomie im wesentlichen dadurch gekennzeichnet, daß der Verankerungsschaft mit zumindest drei außen liegenden, mit der Innenseite eines Röhrenknochens in Eingriff bringbaren Verankerungskanten ausgebildet ist.

Bei dieser Ausbildung kann das Implantat mit seinem Schaft besonders leicht in den Markraum des Knochens eingeführt werden, wobei die Verankerungskanten an der Knocheninnenseite anliegen und eine drehfeste Fixierung des Implantats ermöglichen. Diese Ausbildung gewährleistet auch, daß die Knochenfragmente ausreichend verschoben und präzise positioniert werden können.

Bei Verwendung des erfindungsgemäßen Implantats zur dynamischen Fixation einer Korrekturosteotomie wird insbesondere durch Vorsehen der Kröpfung zwischen dem Befestigungsabschnitt und dem Verankerungsabschnitt eine größere Versetzung zwischen durchtrennten Knochenteilen bzw. -fragmenten möglich, so daß mit einem einzigen Schnitt größere Korrekturen möglich werden, für welche bisher beispielsweise mehrfache Schnitte erforderlich waren. Weiters wird es durch Verwendung des erfindungsgemäßen Implantats möglich, durch Vorsehen des Schnitts entfernt von der Basis neben einer entsprechend der Größe der Kröpfung erzielbaren, großeren Versetzung und somit besseren Korrektur auch unmittelbar eine entsprechend verbesserte Rotationsmöglichkeit der Gelenksfläche zur Verfügung zu stellen, so daß wiederum durch Vorsehen eines einzigen Schnitts gleichzeitig sowohl eine Versetzung in gegenüber dem bisher bekannten Stand der Technik erhöhtem Ausmaß als auch eine Rotation der Gelenkfläche erzielbar ist, wofür gemäß dem bisher bekannten Stand der Technik üblicherweise zwei Schnitte mit entsprechend erhöhtem, operativem Aufwand erforderlich waren.

Durch Einsatz des erfindungsgemäßen Implantats mit einem in Längsrichtung steifen Verankerungsschaft, welcher vorzugsweise mit Verankerungskanten zur Festlegung an der Innenseite eines Röhrenknochens versehen ist, kann mit Hilfe einer minimal-invasiven Technik auch der Operationsvorgang verkürzt und vereinfacht werden, da nach Vorsehen einer verkürzten Schnittlänge im Bereich des zu durchtrennenden Knochens und nach einem Durchtrennen bzw. Schneiden desselben ein Aufweiten des Schnitts beispielsweise durch ein Einsetzen bzw. Einbringen des Verankerungsschafts zwischen die Knochenfragmente erfolgt, wonach nach einem Aufweiten des Schnitts der Verankerungsschaft unmittelbar in den Röhrenknochen eingesetzt bzw. eingetrieben wird, welcher über die vorzugsweise vorgesehenen Verankerungskanten unmittelbar fest im Röhrenknochens verankert wird, so daß zusätzliche, aufwendige Schritte zur Festlegung des Implantats neben der einfachen Festlegung über die Spongiosaschraube im Bereich des Befestigungsabschnitts nicht erforderlich sind.

Für eine besonders einfache Herstellung und zur Erzielung eines entsprechend stabilen Implantats ist gemäß einer weiters bevorzugten Ausführungsform vorgesehen, daß der Befestigungsabschnitt, die Kröpfung und der Verankerungsabschnitt einstückig ausgebildet sind.

Gemäß einem weiteren, bevorzugten Merkmal der Erfindung kann ein im Querschnitt T-förmiger Verankerungsschaft mit spitz zulaufendem Einschlagende vorgesehen sein, der aus einer ebenen, klingenförmigen Ankerplatte und einem von dieser abstehenden, zum Einschlagende hin spitz zulaufenden Steg besteht, wobei die Seitenränder der Ankerplatte und der obere Rand des Steges als Verankerungskanten ausgebildet sind.

Erfindungsgemäß kann bevorzugt mindestens eine der Verankerungskanten zumindest im Bereich des Einschlagendes geschärft sein bzw. messerförmig ausgebildet sein.

Weiters kann der Versatz zwischen der Ankerplatte und dem Befestigungsabschnitt, d.h. die Höhe der Kröpfung, so gewählt werden, daß sie dem gewünschten Ausmaß der Lateralisierung des Knochenfragmentes angepaßt ist.

Gemäß einem weiteren, bevorzugten Merkmal der Erfindung kann das Loch in dem Befestigungsabschnitt als zylindrische Bohrung ausgebildet sein, die zusammen mit dem kopfnahen, zylindrischen Ende der Spongiosaschraube eine winkelstabile Verbindung zwischen Spongiosaschraube und Implantat bildet. Dadurch ist auch die Winkelstabilität zwischen den Knochenfragmenten gewährleistet.

Für eine besonders einfache Festlegung des Befestigungsabschnitts ist das erfindungsgemäße Implantat bevorzugt so ausgebildet, daß das Loch des Befestigungsabschnittes als zylindrische Bohrung ausgebildet ist, die zusammen mit dem kopfnahen, zylindrischen Ende der Spongiosaschraube eine winkelstabile Verbindung zwischen Spongiosaschraube und Implantat bildet.

Zur Verbesserung des Knochenbildungswachstums nach Einsetzen des Implantats und somit zur Verbesserung des Heilungsfortschritts als auch eine vereinfachte Handhabung beim Einbringen in das Innere des Röhrenknochens wird gemäß einer weiters bevorzugten Ausführungsform vorgeschlagen, daß die Kröpfung in ihren seitlichen Bereichen mit einer Verjüngung bzw. Taillierung ausgebildet ist. Durch Vorsehen einer derartigen Verjüngung bzw. Taillierung läßt sich auch das Implantat einfach nach einem Zeitraum von beispielsweise 3 bis 4 Monaten wiederum entnehmen, wobei insbesondere im Bereich der Taillierung eine ausreichende Knochenbildung zur Festigung zwischen den einzelnen Knochenteilen bzw. -fragmenten sichergestellt werden kann.

Für eine besonders einfache Herstellung wird gemäß einer weiters bevorzugten Ausführungsform vorgeschlagen, daß es einstückig aus Metall gefertigt ist. Derartige Metalle bzw. metallische Legierungen, welche für einen Einsatz als Implantate bereits seit längerem erprobt sind, sind bekannt. Insbesondere für den Fall, daß zur Erhöhung der Stabilität der Verbindung zwischen den durchtrennten Knochenteilen eine Entfernung des Implantats nicht vorgenommen werden soll, wird gemäß einer weiters bevorzugten Ausführungsform der Erfindung vorgeschlagen, daß es aus resorbierbarem Kunststoff gefertigt ist.

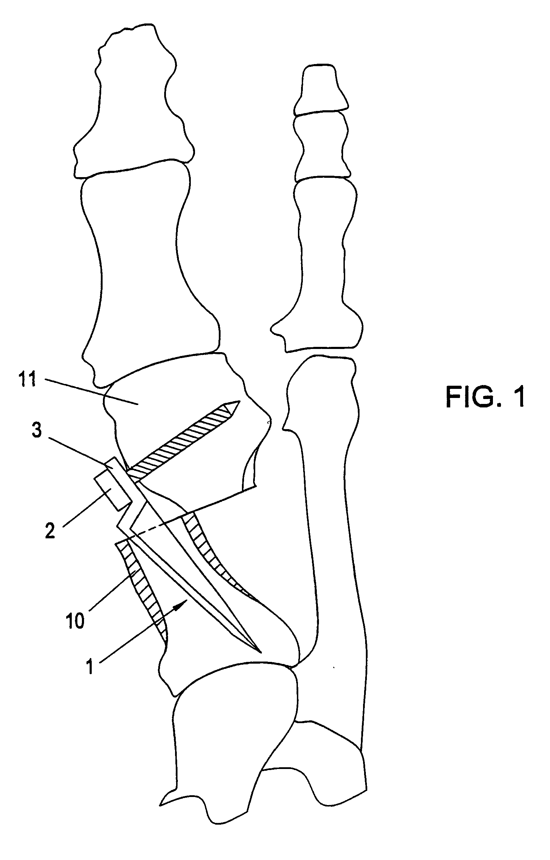

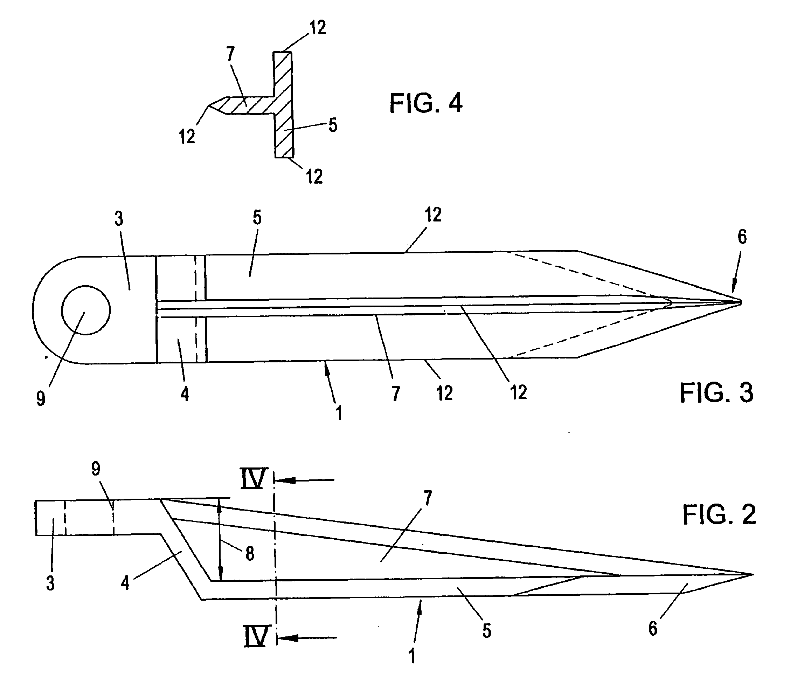

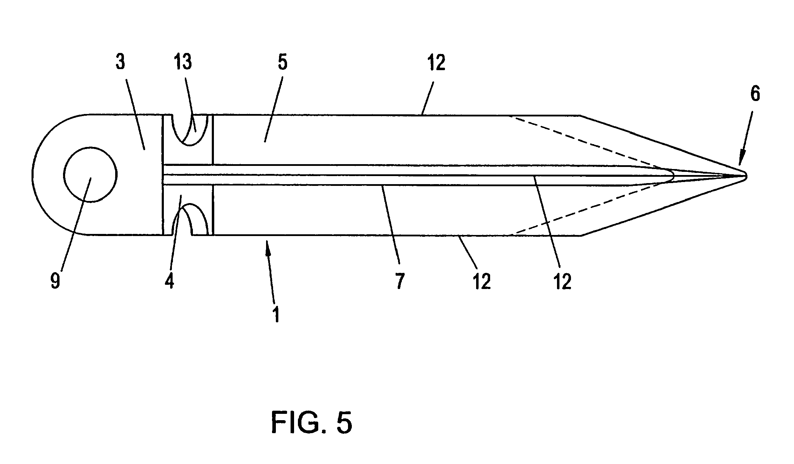

Nachstehend wird die Erfindung an Ausführungsbeispielen anhand der Zeichnungen näher erläutert. In den Zeichnungen zeigt Fig. 1 die Osteosynthese mit einem erfindungsgemäßen Implantat zur Korrektur eines Hallux valgus. Fig. 2 zeigt ein erfindungsgemäßes Implantat von der Seite. Fig. 3 ist eine Draufsicht auf das Implantat der Fig. 2. Fig. 4 stellt einen Querschnitt durch das Implantat der Fig. 2 entlang der Linie IV-IV dar. Fig. 5 stellt eine Draufsicht ähnlich zu Fig. 3 auf eine abgewandelte Ausführungsform eines erfindungsgemäßen Implantats dar.

Eine Möglichkeit der operativen Korrektur des Hallux valgus besteht darin, daß das fehlgestellte Metatarsale I durchtrennt und in korrigierter Stellung fixiert wird. Fig. 1 zeigt eine solche Fixation mit Hilfe des Implantats gemäß Fig. 2. Nach durchgeführter Durchtrennung des Knochens wird der Verankerungsschaft 1 mit dem geschärften Einschlagende so weit in den Markraum des einen Knochenfragmentes 10 eingeschlagen, bis die Kröpfung in Höhe der Osteotomie zu liegen kommt. Dann wird das zweite Knochenfragment 11 mittels einer Spongiosaschraube 2 am Befestigungsabschnitt 3 fixiert. Das Implantat ermöglicht eine in der frontalen und transversalen Ebene sowie in der Rotation stabile Korrekturosteotomie, die in axialer Richtung eine dynamische Kompression der Knochenfragmente erlaubt. Das Implantat zeichnet sich durch hohe statische Stabilität aus. Ein Brechen beim Explantieren ist nicht zu befürchten.

Fig. 2 zeigt eine Ausführungsform des Implantats von der Seite. Das Implantat besteht aus einem Verankerungsschaft 1, der über eine Kröpfung 4 mit einem plattenförmigen Befestigungsabschnitt 3 verbunden ist. Der Verankerungsschaft 1 besteht aus einer ebenen, klingenförmigen Ankerplatte 5 und einem von dieser nach oben abstehenden, zum Einschlagende 6 hin nach vorne spitz zulaufenden Steg 7, wodurch sich die in Fig. 4 gezeigte T-Form des Querschnittes ergibt. Die Seitenränder der Ankerplatte und der obere Rand des Steges sind als Verankerungskanten 12 vorgesehen, die mit der Innenseite des Röhrenknochens in Eingriff zu bringen sind. Die Ankerplatte 5 und der Befestigungsabschnitt 3 liegen auf im wesentlichen parallelen Ebenen. Die Höhe 8 der Kröpfung 4 kann so gewählt sein, daß sie der gewünschten Verschiebung des Knochenfragmentes angepaßt ist.

Der Befestigungsabschnitt 3 weist ein Loch 9 zur Aufnahme einer Schraube 2 auf. Dieses Loch kann als zylindrische Bohrung ausgebildet sein, die mit dem kopfnahen, zylindrischen Ende einer Spongiosaschraube in Eingriff kommt und dabei die gewünschte winkelstabile Verbindung zwischen Schraube und Implantat ermöglicht.

In einer weiteren Ausführungsform des Implantats kann am hinteren Ende des Verankerungsschaftes 1 ein gegenüber der Ankerplatte 5 weiter nach oben versetzter Befestigungsabschnitt vorgesehen sein, wobei die Kröpfung 4 über den Keil nach oben vorsteht.

In Fig. 5 ist eine abgewandelte Ausführungsform dargestellt, wobei die Kröpfung 4 im Bereich ihrer Seitenkanten bzw. Seitenränder jeweils mit einer Taillierung bzw. Verjüngung oder Verengung 13 ausgebildet ist. Im Bereich dieser Verjüngung bzw. Taillierung 13 kann somit zwischen den zueinander versetzten Knochenteilen bzw. -fragmenten 10 und 11 eine verbesserte Knochenbildung und somit eine verbesserte Stabilisierung zwischen den Fragmenten 10 und 11 erzielt werden, wobei durch die Taillierung 13 auch die Handhabung und die nachträgliche Entfernung des Implantats verbessert bzw. vereinfacht wird.

Das Implantat kann aus Metall oder aus einem resorbierbaren Kunststoff gefertigt sein.

## Patentansprüche

1. Implantat zur dynamischen Fixation einer Korrekturosteotomie, wobei ein Befestigungsabschnitt (3) mit einem Loch (9) zur Aufnahme einer Spongiosaschraube über eine Kröpfung (4) mit einem insbesondere seitlich versetzten, langgestreckten Verankerungsabschnitt (1) verbunden ist, der in Längsrichtung als steifer Verankerungsschaft (1) ausgebildet ist, **dadurch gekennzeichnet, daß** der Verankerungsschaft (1) mit zumindest drei außen liegenden, mit der Innenseite eines Röhrenknochens in Eingriff bringbaren Verankerungskanten (12) ausgebildet ist.

2. Implantat nach Anspruch 1, **dadurch gekennzeichnet, daß** der Befestigungsabschnitt (3), die Kröpfung (4) und der Verankerungsabschnitt (1) einstückig ausgebildet sind.

3. Implantat nach Anspruch 1 oder 2, **dadurch gekennzeichnet, daß** ein im Querschnitt T-förmiger Verankerungsschaft mit spitz zulaufendem Einschlagende (6) vorgesehen ist, der aus einer ebenen, klingenförmigen Ankerplatte (5) und einem von dieser abstehenden, zum Einschlagende hin spitz zulaufenden Steg (7) besteht, wobei die Seitenränder der Ankerplatte (5) und der obere Rand des Steges (7) als Verankerungskanten (12) ausgebildet sind.

4. Implantat nach Anspruch 1, 2 oder 3, **dadurch gekennzeichnet, daß** das Loch (9) des Befestigungsabschnittes (3) als zylindrische Bohrung ausgebildet ist, die zusammen mit dem kopfnahen, zylindrischen Ende der Spongiosaschraube (2) eine winkelstabile Verbindung zwischen Spongiosaschraube und Implantat bildet.

5. Implantat nach einem oder mehreren der vorhergehenden Ansprüche, **dadurch gekennzeichnet, daß** mindestens eine Verankerungskante (12) zumindest im Bereich des Einschlagendes messerförmig ausgebildet sind.

6. Implantat nach einem oder mehreren der vorhergehenden Ansprüche, **dadurch gekennzeichnet, daß** die Kröpfung (4) in ihren seitlichen Bereichen mit einer Verjüngung bzw. Taillierung (13) ausgebildet ist.

7. Implantat nach einem oder mehreren der vorhergehenden Ansprüche, **dadurch gekennzeichnet, daß** es einstückig aus Metall gefertigt ist.

8. Implantat nach einem oder mehreren der Ansprüche 1 bis 6, **dadurch gekennzeichnet, daß** es aus resorbierbarem Kunststoff gefertigt ist.

## Claims

1. An implant for the dynamic fixation of a corrective osteotomy, wherein a fixing section (3) including a hole (9) for the reception of a spongiosa screw is connected via a crank (4) with a particularly laterally offset, elongated anchoring section (1) designed in the longitudinal direction as a rigid anchoring shaft (1), **characterized in that** the anchoring shaft (1) is designed to include at least three external anchoring edges (12) capable of engaging the inner side of a long bone.

2. An implant according to claim 1, **characterized in that** the fixing section (3), the crank (4) and the anchoring section (1) are designed in one piece.

3. An implant according to claim 1 or 2, characterize in that an anchoring shaft having a T-shaped cross section and a tapered drive-in end (6) is provided, which is comprised of a plane, blade-shaped anchoring plate (5) and a web (7) projecting from the same and tapering towards the drive-in end, the side edges of the anchoring plate (5) and the upper edge of the web (7) being designed as anchoring edges (12).

4. An implant according to claim 1, 2 or 3, **characterized in that** the hole (9) provided in the fixing section (3) is designed as a cylindrical bore which, together with the head-near, cylindrical end of the spongiosa screw, forms an angularly stable connection between the spongiosa screw and the implant.

5. An implant according to one or several of the preceding claims, **characterized in that** at least one of the anchoring edges (12) is knife-shaped, at least in the legion of the drive-in end.

6. An implant according to one or several of the preceding claims, **characterized in that** the crank (4) is designed to include a diminution or reduction (13) in its lateral regions.

7. An implant according to one or several of the preceding claims, **characterized in that** is designed in one piece of metal.

8. An implant according to one or several of claims 1 to 6, **characterized in that** it is made of resorbable plastics.

## Revendications

1. Implant pour fixation dynamique d'une ostéotomie corrective, un segment de fixation (3) comportait un trou (9) pour recevoir une vis pour tissu spongieux étant relié par un coude (4) à un segment d'ancrage (1) allongé, en particulier, décalé latéralement qui est formé dans le sens longitudinal en tant que tige d'ancrage rigide (1), **caractérisé en ce que** la tige d'ancrage (1) est constituée d'au moins trois arêtes d'ancrage (12) pouvant être mises en prise avec la face interne d'un os long.

2. Implant selon la revendication 1, **caractérisé en ce que** le segment de fixation (3), le coude (4) et le segment d'ancrage (1) sont formés d'un seul tenant.

3. Implant selon la revendication 1 ou 2, **caractérisé en ce qu'**une tige d'ancrage présentant une section en forme de T est dotée d'une extrémité de coupe se terminant en pointe (6), qui est constituée d'une plaque d'ancrage plane en forme de lame (5) et d'un étai (7) partant de celle-ci, convergeant en pointe vers l'extrémité de coupe, les bords latéraux de la plaque d'ancrage (5) et du bord supérieur de l'étai (7) étant configurés en tant qu'arêtes d'ancrage (12).

4. Implant selon la revendication 1, 2 ou 3, **caractérisé en ce que** le trou (9) du segment de fixation (3) est configuré comme un alésage cylindrique qui forme, conjointement avec l'extrémité cylindrique à proximité de la tête de la vis pour tissu spongieux (2), une liaison stable au niveau angulaire entre la vis pour tissu spongieux et l'implant.

5. Implant selon une ou plusieurs des revendications précédentes, **caractérisé en ce qu'**au moins une arête d'ancrage (12) est en forme de couteau au moins dans la zone de l'extrémité de coupe.

6. Implant selon une ou plusieurs des revendications précédentes, **caractérisé en ce que** le coude (4) est formé dans ses zones latérales, d'un rétrécissement ou d'une découpe (13).

7. Implant selon une ou plusieurs des revendications précédentes, **caractérisé en ce qu'**il est en métal d'un seul tenant.

8. Implant selon une ou plusieurs des revendications 1 à 6, **caractérisé en ce qu'**il est constitué de matière plastique résorbable.
